# EUROPEAN PATENT SPECIFICATION

(11) **EP 2 303 152 B1**
(45) Date of publication and mention of the grant of the patent: **23.01.2019**
(21) Application number: 09758970.9
(22) Date of filing: 18.05.2009
(51) Int. Cl.: A61B 17/32, A61B 17/3207, A61B 18/14

(54) **ABRASIVE NOSE CONE WITH EXPANDABLE CUTTING AND SANDING REGION FOR ROTATIONAL ATHERECTOMY DEVICE**
ABRASIVER NASENKONUS MIT EXPANDIERBARER SCHNEIDE- UND SCHLEIFREGION FÜR EINE ROTIERENDE ATHEREKTOMIE-VORRICHTUNG
CÔNE À NEZ ABRASIF AVEC RÉGION DE DÉCOUPE ET DE PONÇAGE EXPANSIBLE POUR UN DISPOSITIF D'ATHÉRECTOMIE ROTATIF

(30) Priority: 05.06.2008 US 59028 P; 14.05.2009 US 466152
(43) Date of publication of application: 06.04.2011
(73) Proprietor: Cardiovascular Systems, Inc., St. Paul, MN 55112 (US)
(72) Inventor: RIVERS, Jody, Elk River MN 55330 (US); WELTY, Ryan, Blaine MN 55449-5592 (US); CAMBRONNE, Matt, Moundsview MN 55112 (US); FRANCHINO, David, C., Madison WI 53718 (US); KALLSEN, Kent, Jeffrey, Jefferson WI 53549 (US); LATHAM, Stephen, Sun Prairie WI 53590 (US)
(74) Representative: Zacco Denmark A/S
(86) International application number: PCT/US2009/044320
(87) International publication number: WO 2009/148809

(56) References cited:
- WO-A2-98/18520
- WO-A2-03/088809
- WO-A2-2005/041748
- US-A- 4 966 604
- US-A- 5 030 201
- US-A- 5 318 576
- US-A1- 2005 149 084
- US-B1- 6 216 043

## Description

### CROSS-REFERENCE TO RELATED APPLICATIONS

The present application claims priority to US patent 2009306690, filed on June 5, 2008 under the title, "CUTTING AND SANDING RIBBON WISK".

### STATEMENT REGARDING FEDERALLY SPONSORED RESEARCH OR DEVELOPMENT

Not Applicable

### BACKGROUND OF THE INVENTION

### FIELD OF THE INVENTION

The present invention is directed generally to expandable abrasive grinding and cutting heads for rotational atherectomy devices.

### DESCRIPTION OF THE RELATED ART

Atherectomy is a non-surgical procedure to open blocked coronary arteries or vein grafts by using a device on the end of a catheter to cut or shave away atherosclerotic plaque (a deposit of fat and other substances that accumulate in the lining of the artery wall). For the purposes of this application, the term "abrading" is used to describe the grinding and/or scraping action of such an atherectomy head.

Atherectomy is performed to restore the flow of oxygen-rich blood to the heart, to relieve chest pain, and to prevent heart attacks. It may be done on patients with chest pain who have not responded to other medical therapy and on certain of those who are candidates for balloon angioplasty (a surgical procedure in which a balloon catheter is used to flatten plaque against an artery wall) or coronary artery bypass graft surgery. It is sometimes performed to remove plaque that has built up after a coronary artery bypass graft surgery.

Atherectomy uses a rotating shaver or other device placed on the end of a catheter to slice away or destroy plaque. At the beginning of the procedure, medications to control blood pressure, dilate the coronary arteries, and prevent blood clots are administered. The patient is awake but sedated. The catheter is inserted into an artery in the groin, leg, or arm, and threaded through the blood vessels into the blocked coronary artery. The cutting head is positioned against the plaque and activated, and the plaque is ground up or suctioned out.

The types of atherectomy are rotational, directional, and transluminal extraction. Rotational atherectomy uses a high speed rotating shaver to grind up plaque. Directional atherectomy was the first type approved, but is no longer commonly used; it scrapes plaque into an opening in one side of the catheter. Transluminal extraction coronary atherectomy uses a device that cuts plaque off vessel walls and vacuums it into a bottle. It is used to clear bypass grafts.

Several devices have been disclosed that perform rotational atherectomy. For instance, U.S. Patent No. 5,360,432, issued on November 1, 1994 to Leonid Shturman, and titled "Abrasive drive shaft device for directional rotational atherectomy" discloses an abrasive drive shaft atherectomy device for removing stenotic tissue from an artery. The device includes a rotational atherectomy apparatus having a flexible, elongated drive shaft having a central lumen and a segment, near its distal end, coated with an abrasive material to define an abrasive segment.

Expandable atherectomy devices have been actively sought in order to gain the advantage of the small retracted position diameter during insertion and placement in the vasculature while allowing the device to achieve an expanded position comprising larger-than-retracted diameter during high-speed rotation.

Examples of such efforts are found in, e.g., U.S. Patent No. 4,966,604 to Reiss; U.S. Patent No. 5,030,201, issued on July 9, 1991 to Palestrant; U.S. Patent No. 5,178,625, issued on January 12, 1993 to Groshong; and U.S. Patent No. 5,376,100, issued on December 27, 1994 to Lefebvre.

Collectively, the five references discussed above disclose atherectomy devices capable of moving cutting elements from a retracted position to an expanded position. In one aspect, the cutting elements include blades with and without abrasive thereon. Certain of these disclosed blades include sharp metal edges while others include flexible plastic cutting elements. Movement from retracted to expanded position may be achieved by mechanical manipulation by the operator using, for example, a central wire through the cutting element with a distal stop thereon which is slidable in response to pulling/pushing force applied by the operator. In some aspects, the distal cutting head is fixed to an axially slidable wire. A variation of this mechanism provides desmodromic wire(s) with a slidable tip and an axial motion stop to allow expansion (bowing) and retraction of the cutting wires. Alternatively, centrifugal force, for example, flexible plastic cutting elements or fibers, is sufficient to achieve the expanded position. The various designs achieve cutting and/or sanding by, for instance, rotating motion driven by a powered means.

Additional expandable cutting means are disclosed in the following references.

U.S. Patent No. 7,291,146, issued on November 6, 2007 to Steinke et al, discloses a radially expandable structure with electrosurgical energy delivery devices attached thereto, for example, electrodes, for engaging the atherosclerotic material.

U.S. Patent No. 5,224,945, issued on July 6, 1993 to Pannek discloses an expandable and compressible atherectomy cutter having a plurality of radially separated and axially joined blades. The distal ends of these blades are joined by a grommet which is slidable on a guide wire, while the proximal ends are fixed in position. Expansion of the blades is achieved by pulling on the guide wire, which includes a stop distal to the grommet.

U.S. Patent No. 5,318,576, issued on June 7, 1994 to Plassche Jr. et al, discloses a cutter of preferably ellipsoidal profile including a plurality of flexible segments. These flexible segments having cutting elements thereon, for example, cusps and mounds, distributed over the outer surface. The cutter is shortened axially to radially expand the flexible segments by use of a guide wire having a distal stop thereon.

U.S. Patent No. 5,556,408, issued on September 17, 1996 to Farhat discloses an expandable and compressible atherectomy cutter including a distal hub and a proximal hub joined by a plurality of resilient blades. The blades are shaped to describe a cutting radius which may be compressed.

U.S. Patent No. 5,766,191, issued on June 16, 1998 to Trerotola discloses a thrombolytic fragmentation device including a wire cage or basket attached to a rotational drive. The wire basket or cage is encased within a catheter and, when released therefrom, automatically conforms to the inner dimension of the vessel lumen. The wire basket or cage is defined in '704 as being made from three to six wires, made from a shape memory material such as nitinol, wherein the undeformed position is the expanded or bowed position. The wires may include abrasive thereon or have a cutting edge. The rotational speed disclosed is relatively low, with the highest disclosed speed being 5,000 rpm.

U.S. Patent No. 6,800,083, issued on October 5, 2004 to Hiblar et al, discloses a compressible atherectomy burr having one or more flexible abrasive disks foldable to be slidably received within a catheter. In another design, a resilient and flexible panel spirals outwardly, forming a generally cylindrical ablation surface.

In several of these references, members span a portion of the atherectomy head. These members are fixed at their proximal and distal ends, and are generally free in the portions between the proximal and distal ends. When the fixed proximal and distal ends of the members are brought toward each other, the members bow and expand radially outward. When rotated about the drive shaft, these radially expanded members can cut, scrap or grind at blockages that are larger in diameter than the rest diameter of the atherectomy head.

However, none of these references disclose a distal nose cone coated with abrasive, a proximal section of which is expandable. In this regard, the most distal taper of the nose cone may be used to open partially or completely occluded vessels, thereby creating a pilot hole. The remainder of the retracted cylindrical and low profile nose cone may then be gradually worked into the occlusion, whereupon the distal tapered section of the nose cone may be pulled back or distally, causing the cutting members to bow outwardly in an expanded position. Rotation of the nose cone in either the retracted or the expanded position facilitates opening of the occlusion. Document WO 03/088809 A2 discloses a rotational intra vascular cutting apparatus with a plurality of distal, radially expandable, abrasive cutting blades.

### BRIEF SUMMARY OF THE INVENTION

Claim 1 defines the invention and the dependent claims disclose the preferred embodiments. One embodiment of the present invention comprises a rotational atherectomy apparatus for abrading tissue, comprising: a flexible, elongated, rotatable drive shaft having a proximal end and a distal end opposite the proximal end; a nose cone operatively attached proximate the distal end of the drive shaft comprising a distal tapered section and a plurality of elongate, flexible members adjacent to the distal tapered section of the drive shaft, each member in the plurality being fixed at both a proximal end and a distal end opposite the proximal end; a proximal mount rotatable with the drive shaft and fixedly connected to the proximal ends of all the flexible members in the plurality; and a distal mount axially separated from the proximal mount and fixedly connected to the distal ends of all the flexible members in the plurality. When the axial separation of the proximal and distal mounts is reduced by pulling the distal tapered section proximally, each member in the plurality bows outward from the drive shaft and expands radially. The axial separation of the proximal and distal mounts may be achieved by an actuator cable or tube through the inner diameter of the drive shaft and in operative communication with the distal tapered section. In the retracted position, the plurality of elongate, flexible members comprise a cylindrical profile while the distal tapered section comprises a proximal cylindrical profile with diameter equivalent to the retracted plurality of elongate, flexible members and a distal conical profile. In the expanded position, the plurality of elongate, flexible members comprise in one embodiment, an at least partially elliptical, symmetric, profile and in another embodiment, an eccentric or asymmetric profile, while the distal tapered section comprises a proximal cylindrical profile with diameter equivalent to the retracted plurality of elongate, flexible members and a distal conical profile. In other words, the distal tapered section's profile remains constant in both the retracted and expanded positions.

One embodiment of the present invention comprises a rotational atherectomy system for abrading tissue, comprising: a guide wire, a catheter having a lumen therethrough and capable of advancement/retraction over the guide wire; a flexible, elongated, rotatable drive shaft having a proximal end and a distal end opposite the proximal end; a nose cone operatively attached proximate the distal end of the drive shaft comprising a distal tapered section and a plurality of elongate, flexible members adjacent to the distal tapered section of the drive shaft, each member in the plurality being fixed at both a proximal end and a distal end opposite the proximal end; a proximal mount rotatable with the drive shaft and fixedly connected to the proximal ends of all the flexible members in the plurality; and a distal mount axially separated from the proximal mount and fixedly connected to the distal ends of all the flexible members in the plurality. When the axial separation of the proximal and distal mounts is reduced by pulling the distal tapered section proximally, each member in the plurality bows outward from the drive shaft and expands radially. The axial separation of the proximal and distal mounts may be achieved by an actuator cable or tube through the inner diameter of the drive shaft and in operative communication with the distal tapered section. In the retracted position, the plurality of elongate, flexible members comprise a cylindrical profile while the distal tapered section comprises a proximal cylindrical profile with diameter equivalent to the retracted plurality of elongate, flexible members and a distal conical profile. In the expanded position, the plurality of elongate, flexible members comprise in one embodiment, an elliptical, symmetric, profile and in another embodiment, an eccentric or asymmetric profile, while the distal tapered section comprises a proximal cylindrical profile with diameter equivalent to the retracted plurality of elongate, flexible members and a distal conical profile. In other words, the distal tapered section's profile remains constant in both the retracted and expanded positions. In addition, the system comprises means for rotating the drive shaft.

An exemplary method for abrading a blockage is described, comprising: advancing a guide wire through a vasculature of a patient to the blockage; advancing a catheter over the guide wire to the blockage; advancing a rotatable drive shaft comprising the inventive nose cone operatively attached proximate the distal end of the drive shaft within the catheter to the blockage; advancing the distal tapered section of the nose cone to the blockage and initiating rotating and/or axial movement of the nose cone; creating a pilot hole through the blockage if necessary; advancing the plurality of elongate, flexible members through the catheter proximate the blockage; and beyond a distal end of the catheter to the blockage, the members being fixedly connected at their proximal ends to a common proximal mount and being fixedly connected at their distal ends to a common distal mount, the proximal and distal mounts being axially separated, the proximal mount being rotatably coupled to the drive shaft; actuating the plurality of elongate, flexible members into an expanded position, wherein the plurality of flexible members bow radially outward from the drive shaft; rotating the drive shaft; abrading the blockage through repeated contact with the plurality of bowed flexible members; stopping the rotation of the drive shaft; returning the plurality of flexible members to the retracted position; withdrawing the plurality of flexible members into the catheter; withdrawing the system from the vasculature of the patient.

Alternate exemplary methods may comprise the flexible members being biased in the bowed expanded position so that the flexible members radially expand upon advancement out of the catheter and radially compress into a retracted position upon withdrawal into the catheter.

### BRIEF DESCRIPTION OF THE SEVERAL VIEWS OF THE DRAWINGS

Figure 1 is a perspective drawing of one embodiment of a rotational atherectomy device.
Figure 2 is a partial cutaway of one embodiment of a drive shaft with nose cone operatively attached proximate the distal end of the drive shaft and in a retracted position.
Figure 3 is a partial cutaway of one embodiment of a drive shaft with nose cone operatively attached proximate the distal end of the drive shaft and in an expanded position.

### DETAILED DESCRIPTION OF THE INVENTION

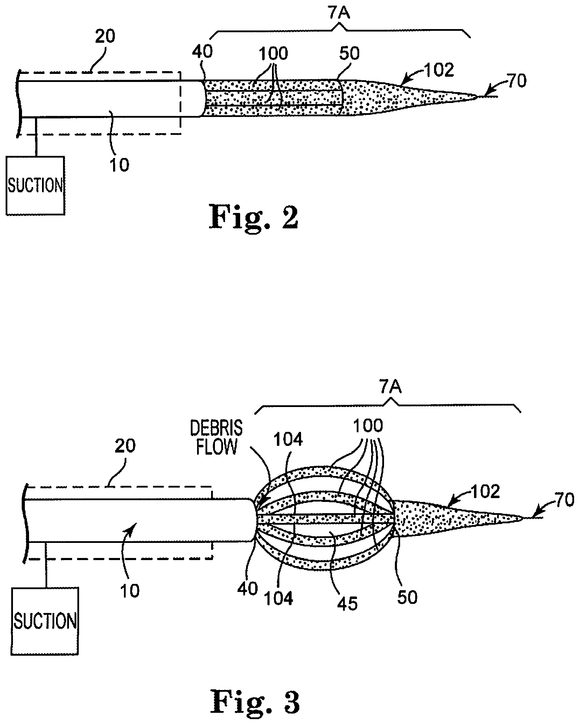

A rotational atherectomy device is disclosed, in which an abrasive nose cone is attached to a rotatable drive shaft. The abrasive head includes a distal tapered section and, proximal thereto, a plurality of elongate, flexible members, such as wires, that are attached at their proximal ends to a proximal mount, and are attached at their distal ends to a distal mount. The proximal and distal mounts and the distal tapered section are rotatably operatively attached to a drive shaft, and rotate with the drive shaft. The proximal and distal mounts are axially separated from each other. As the axial spacing decreases, the flexible members bow radially outward, actuated by an actuator device, e.g., an actuator cable or the equivalent operatively placed in the lumen of the drive shaft and attached to the distal tapered section. A proximal force applied to the actuating device may cause the distal tapered section to move proximally, causing a bowing in the flexible members to an expanded position that varies in a directly proportional manner with the degree of proximal force applied to the actuating device up to a maximum expanded diameter that may be achieved. In this embodiment, the flexible members are biased in a relatively straightened configuration to comprise the cylindrical profile shown in Figure 2.

In alternate embodiments, the flexible members may be biased in an outwardly bowed configuration and held in a retracted position by the catheter lumen. When the flexible members are advanced beyond the distal end of the catheter lumen, the flexible members are released and expand axially outwardly. Returning the flexible members proximally into the confines of the catheter lumen results in the flexible members retracting into a retracted position. Those skilled in the art will recognize that automatically sizes the flexible members to the blockage or to the vessel to be cleaned, without user intervention.

Prior to use, the drive shaft, the proximal mount, the flexible members and the distal mount are all contained within the catheter's lumen, and all surround the guide wire. First, a user feeds the guide wire is through the vasculature of the patient to the blockage. Next, the user advances the catheter and its contents over the guide wire to the blockage. The catheter has a generally smooth exterior and does not damage any of the blood vessels as it is advanced along the guide wire. Once the distal end of the catheter is positioned at or near the blockage, the user advances the drive shaft with respect to the catheter (and/or, equivalently, retracts the catheter with respect to the drive shaft). The axial pressure from the drive shaft pushes the abrasive nose cone out the distal end of the catheter, with the drive shaft pushing on the proximal mount, the proximal mount pushing on the flexible members, and the flexible members pushing on the distal mount. Note that the flexible members, e.g., wires, can support this relatively small amount of axial pressure, and can facilitate pushing the nose cone out of the catheter.

In the embodiment wherein the flexible members are biased in an expanded position and once the flexible members are pushed out of the catheter, the catheter wall no longer constrains them radially, and they are free to expand radially to automatically press against the sides of the occlusion without further operator intervention. A benefit of such an abrasive head is that the radial expansion of the flexible members is automatic, and does not require any additional steps from the user or any additional elements in the catheter. Furthermore, the radial expansion does not rely on centrifugal force, so that the expansion may occur at relatively low rotational speeds or at rest. The proximal mount, flexible members, distal mount and tapered distal section all rotate with the drive shaft. The tapered distal section opens, when needed, a pilot hole sufficient in diameter for the flexible members to begin to operate in gradually expanding positions, the radially expanded flexible members cut, scrape and/or grind away at the blockage in the vessel. As the blockage is abraded, the flexible members grow radially to the proper size automatically, again without any intervention from the user. Once the blockage is fully abraded, the rotation of the drive shaft is reduced or stopped, and the user retracts the drive shaft with respect to the catheter (and/or, equivalently, the user advances the catheter with respect to the drive shaft). The drive shaft pulls the proximal mount inside the catheter, the proximal mount pulls the flexible members inside the catheter and radially compresses them while doing so for removal.

In the embodiment wherein the flexible members are biased in the retracted position, proximal force applied to the actuator by the operator causes the distal tapered section to move proximally, in turn causing the flexible members to bow radially outwardly into an expanded position that is controlled by the operator's proximal force on the actuating device. Advantages of this embodiment comprise the operator's ability to actively control the diameter of the nose cone's flexible members when actuated to an expanded position. In addition, as above, centrifugal force is not used, nor is it required, to actuate the flexible members to an expanded position.

Once the blockage is fully abraded, the rotation of the drive shaft is reduced or stopped, and the user retracts the drive shaft with respect to the catheter (and/or, equivalently, the user advances the catheter with respect to the drive shaft) in accordance with the nose cone embodiments described above.

The above paragraphs are merely a summary of the disclosure, and should not be construed as limiting in any way. A more detailed description follows.

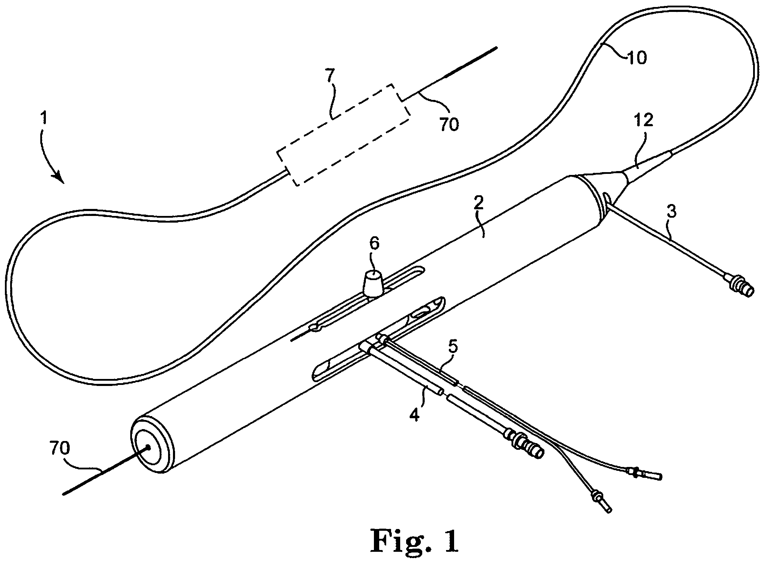

FIG. 1 is a schematic drawing of a typical rotational atherectomy device. The device includes a handle portion 2, an elongated, flexible drive shaft, and an elongated catheter 10 extending distally from the handle portion 2. The drive shaft transmits torque from a controlling proximal end 12, at or near the handle portion 2, to an abrasive head 7 at or near a distal end of the drive shaft. The catheter 10 has a lumen in which most or all of the length of the drive shaft is disposed. The drive shaft also contains an inner lumen, permitting the drive shaft to be advanced and rotated over a guide wire 70. A fluid supply line 3 may be provided for introducing a cooling and lubricating solution (typically saline or another biocompatible fluid) into the catheter 10.

The handle 2 may contain a turbine (or similar rotational drive mechanism) for rotating the drive shaft at high speeds. The handle 2 typically may be connected to a power source, such as compressed air delivered through a tube 4. A pair of fiber optic cables 5 may also be provided for monitoring the speed of rotation of the turbine and drive shaft. Details regarding such handles and associated instrumentation are well known in the industry, and are described, e.g., in United States Patent No. 5,314,407, titled "Clinically practical rotational angioplasty system", issued on May 24, 1994 to David C. Auth et al. The handle 2 also desirably includes a control knob 6 for advancing and retracting the turbine and drive shaft with respect to the catheter 10 and the body of the handle.

There are several ways to rotate the drive shaft during operation. In most cases, a motor is attached to the drive shaft at or near the proximal end of the drive shaft. A suitable control system for such a motor is disclosed in U.S. Patent Application No. 10/272,126, to Shturman et al, titled "Control system for rotational angioplasty device", and published on June 26, 2003 as U.S. Patent Application Publication No. US 2003/0120296 A1.

Other methods of drive shaft rotation are possible as well. For instance, the user can directly rotate the drive shaft by hand. As another example, the user can turn a crank connected to the drive shaft. As a further example, the user can turn a crank, with the crank driving a geared system that scales up the rotational speed of the drive shaft by a particular factor over the rotational speed of the crank. Other suitable rotation-producing systems are possible as well.

The present application is directed mainly to a design of the abrasive head 7. In this respect, many or all of the other elements of the known atherectomy device of FIG. 1 may be used with the present disclosed head design, including the catheter 10, the guide wire 70, and the handle 2 along with its controls and its inputs and outputs.

Note that Figure 1 shows the guide wire 70 extending beyond the abrasive head 7. Typically, the guide wire 70 is the first element of the atherectomy device 1 inserted into the blood vessel. Being much thinner than the catheter 10, the guide wire 70 is much easier to navigate through the vasculature of the patient from the insertion point to the blockage. Once the guide wire 70 reaches the blockage, the catheter 10 may be advanced along the guide wire 70 until the abrasive head 7 is suitably located at or near the blockage.

Once the abrasive head 7 is placed, there are several options available for the guide wire. In most cases considered herein, the guide wire 70 is left in place and extends beyond the distal end of the abrasive head 7. This helps provide stability for the distal mount during use. In other cases, the guide wire 70 may be retracted partially into the catheter 10, so that it does not extend into or beyond the abrasive head. In still other cases, the guide wire 70 may be retracted completely from the catheter 10. After the blockage has been removed, retracting the catheter 10 does not require the use of a guide wire 70, since the retraction involves pulling the catheter 10 from the insertion point and does not require any particular navigation through the vasculature of the patient.

Figures 2 and 3 show an exemplary abrasive nose cone 7A, operatively attached to the distal end of the drive shaft 10 which is disposed, axially translatable and rotatable within catheter 20. The abrasive nose cone 7A comprises a plurality of flexible, elongate members 100, which can be wires or strips of suitable cutting or grinding material. The wires may have a circular cross-section, or may have an asymmetric cross section that may enhance their cutting or scraping abilities. For instance, an asymmetric cross-section may be a half-circle, triangular, rectangular, square, and/or may include one or more corners. In Figure 2, the flexible members 100 are radially compressed and axially extended, as they are when inside the lumen of catheter 100 and in a retracted position. In Figure 3, the flexible members 100 are axially compressed and radially expanded, as they are when outside the catheter and during use in an expanded position.

As illustrated, the proximal ends of the members 100 are all attached to a proximal mount 40. In some cases, some or all of the members 100 may attach to each other before attaching to the proximal mount 40. In other cases, the members 100 may attach only to the proximal mount 40 and do not attach to each other. The proximal mount 40 is mechanically and rotatably coupled to the drive shaft 10 in most cases being at or near the distal end of the drive shaft. As the drive shaft 10 rotates, the proximal mount 40 rotates as well. Similarly, if the drive shaft 10 is axially translated within the catheter 20, the proximal mount 40 follows. The proximal mount 40 may be made integral with the drive shaft 10, or may be made separately from the drive shaft 10 and attached to the drive shaft 10. In many cases, the drive shaft 10 is formed as one or more helically coiled wires, and the proximal mount 40 is a solid structure operatively attached to the distal end of the drive shaft 10.

In the same manner that the proximal ends of the flexible members 100 are attached to a common proximal mount 40, the distal ends of the flexible members are attached to a common distal mount 50 which is operatively attached to, or integral with, the distal tapered section 102. In some cases, some or all of the members 100 may attach to each other before attaching to the distal mount 50 which is operatively attached to, or integral with, the distal tapered section 102. In other cases, the members 100 may attach only to the distal mount 50 and do not attach to each other. In all cases, the distal tapered section 102 is connected with the drive shaft 10 via the distal mount 50, the flexible members 30A and the proximal mount 40. Therefore, the distal tapered section 102 translates and rotates in concert with the drive shaft 10, the flexible members 100, and the proximal and distal mounts 40, 50.

In many cases, the guide wire 70 passes through the proximal and distal mounts 40, 50 and through a lumen 45 (shown in Fig. 3) within the flexible members 100 and keeps the proximal and distal mounts 40, 50 and the flexible members 100, in certain embodiments, roughly centered along the rotational axis of the drive shaft 10 as the drive shaft 10 rotates. Such stability may be useful at the high rotational speeds, e.g., 20,000 rpm or higher, that are generally required for rotational atherectomy.

As shown in Fig. 3, the flexible members 100 comprise an expanded elliptical profile wherein each flexible member 100 is arranged circumferentially and angularly equidistant from the adjacent flexible member 100. This arrangement results in a symmetric plurality of flexible members 100 with a center of mass on the axis of rotation of the drive shaft. Accordingly, high-speed rotation of the expanded flexible members 100 will achieve a rotational (and abrasive/cutting) diameter that is equivalent to the resting diameter of the expanded flexible members.

Alternate embodiments may comprise the flexible members 100 having an at least partially elliptical profile wherein a portion of the plurality of the flexible members 100 comprise an elliptical profile as described above while the remainder of the flexible members 100 are arranged with larger, or smaller, distances from one flexible member 100 to the next adjacent flexible member 100. This alternative arrangement results in an asymmetric plurality of flexible members 100 with a center of mass for the flexible members that is not on the drive shaft 10 axis of rotation. As a result, high-speed rotation of the abrasive nose cone will result in an eccentric, or orbital, motion as is well described in U.S. Pat 6,494,890 to Shturman. The advantage of an eccentric, or asymmetric, plurality of flexible members 100 is that the rotational (and abrasive/cutting) diameter of the expanded plurality of flexible members 100 is larger than the resting diameter of the expanded plurality of flexible members 100. This, in turn, allows a lower profile abrasive element, i.e., abrasive nose cone 7A, to be inserted into the patient's vasculature which, *inter alia,* reduces trauma.

Alternative mechanisms may be employed to achieve offset of the center of mass from the rotational axis of the drive shaft. For example, at least part of one or more of the plurality of flexible members 100 may comprise a material that is of a density that is more, or less, dense than the density of the remaining flexible members 100. Those skilled in the art will now recognize the utility in such an arrangement, as well as the large number of permutations that may be engineered into the present invention. Each such resulting embodiment is well within the scope of the present invention.

The distal tapered section 102 is located distal to, and adjacent, the flexible members 100 and connected thereto by distal mount 50 as described above. The distal tapered section 102 comprises a proximal fixed cylindrical profile and a distal fixed conical profile. The distal tapered section 102 further comprises a lumen therethrough (not shown) to allow axial translation over the guide wire 70. Since the distal mount 50 is operatively attached to, or integral with, the distal tapered section 102, the distal mount 50 is also free to axially translate, or slide, along the guide wire 70.

The exterior surface of the flexible members 100 and the distal tapered section 102 may be coated, in whole or in part(s), with an abrasive material. The abrasive material may be any suitable material, such as diamond powder, fused silica, titanium nitride, tungsten carbide, aluminum oxide, boron carbide, or other ceramic materials. In some cases, the abrasive material includes diamond chips or diamond dust particles, attached directly to the exterior of the flexible members 100 and the distal tapered section 102 by a suitable binder. The material may be attached using well known techniques, such as conventional electroplating or fusion technologies (see, for example, U.S. Patent No. 4,018,576. Alternately, the exterior surface of the flexible members 100 and/or the distal tapered section 102 may be mechanically or chemically roughened, and/or may be etched or cut, as with a laser, to provide a sanding, grinding, cutting, or slicing surface.

The flexible members 100 comprise sharp cutting side edges 104 along both sides to provide a cutting surface. These cutting side edges 104 are exposed only when the nose cone is in an expanded position as in Fig 2. Otherwise, when in a retracted position as in Fig. 1, the cutting edges 104 are not exposed.

Thus, the nose cone allows for grinding of occluding material along abrasive surface of the distal tapered section 102 and the retracted flexible members 100. In addition, the expanded flexible members 100 allow for cutting of occluding material.

As provided in the Figures, a suction means as is well known in the art may be provided to enable a debris flow proximally through the lumen of the drive shaft and away from the occlusion. Alternatively, a distal protection device, also well known in the art, may be employed to capture the debris generated during the atherectomy.

The description of the invention and its applications as set forth herein is illustrative and is not intended to limit the scope of the invention. Variations and modifications of the embodiments disclosed herein are possible and practical alternatives to and equivalents of the various elements of the embodiments would be understood to those of ordinary skill in the art upon study of this patent document. These and other variations and modifications of the embodiments disclosed herein may be made without departing from the scope of the invention.

## Claims

1. A rotational atherectomy apparatus for abrading occluding material within the lumen of a blood vessel, comprising:
a flexible, elongated, rotatable drive shaft (10) having a proximal end (12) and a distal end opposite the proximal end (12);
a nose cone (7A) comprising:
a plurality of elongate, flexible members (100) proximate the distal end of the drive shaft (10), each member (100) in the plurality being fixed at both a proximal end and a distal end opposite the proximal end and comprising a biased retracted position having a cylindrical profile, an exterior surface having abrasive coating thereon, and sharp cutting side edges (104), wherein the elongate, flexible members (100) are rotatable with the drive shaft (10); a proximal mount operatively connected with and rotatable with the drive shaft and fixedly connected to the proximal ends of all the flexible members in the plurality of elongate, flexible members (100);
a distal mount axially separated from the proximal mount and fixedly connected to the distal ends of all the flexible members (100) in the plurality and rotatable with the drive shaft (10) and the plurality of elongate, flexible members (100); and
a distal tapered section (102) comprising a proximal fixed cylindrical profile and a distal fixed conical profile, the distal tapered section (102) attached to the distal mount and rotatable with the drive shaft (10) and the plurality of elongate, flexible members (100);
wherein when the axial separation of the proximal and distal mounts is reduced, each member (100) in the plurality bows outward from the drive shaft (10) and expands radially away from the biased retracted position to an expanded position, the flexible members (100) thereby comprising an expanded elliptical profile, **characterised in that**
the drive shaft (10) comprises an axis of rotation and the plurality of flexible members (100) further comprises a center of mass offset from the axis of rotation through an asymmetrical arrangement of the flexible members around the drive shaft or one or more flexible members comprising a material of a different density than the remaining flexible members;
such that the expanded plurality of flexible members (100) has a resting expanded diameter and a rotational cutting diameter, the latter achieved during high-speed rotation, wherein the rotational cutting diameter is larger than the resting expanded diameter due to orbital or eccentric movement of the rotating cutter;
**in that** the nose cone (7A) is an abrasive nose cone;
and **in that** the distal tapered section (102) has a lumen therethrough.

2. The rotational atherectomy apparatus of claim 1, wherein the plurality of flexible members (100) comprises flexible members are arranged asymmetrically.

3. The rotational atherectomy apparatus of claim 1, wherein the flexible members (100) are wires.

4. The rotational atherectomy apparatus of claim 3, wherein at least one of the wires has a circular cross-section.

5. The rotational atherectomy apparatus of claim 3, wherein at least one of the wires has a non-circular cross-section.

6. The rotational atherectomy apparatus of claim 1, further comprising an actuating device which allows the operator to control the reduction of axial separation of the proximal and distal mounts.

7. The rotational atherectomy apparatus of claim 1, wherein the flexible members (100) are mounted in a parallel array and are spaced angularly apart from each other.

## Patentansprüche

1. Rotierende Atherektomievorrichtung zum Abreiben eines Okklusionsmaterials innerhalb des Lumens eines Blutgefäßes, umfassend:
eine flexible, längliche, drehbare Antriebswelle (10), die ein proximales Ende (12) und ein dem proximalen Ende (12) gegenüberliegendes distales Ende aufweist;
einen Nasenkonus (7A) umfassend:
eine Vielzahl von länglichen, flexiblen Elementen (100) in der Nähe des distalen Endes der Antriebswelle (10), wobei jedes Element (100) in der Vielzahl sowohl an einem proximalen Ende als auch an einem dem proximalen Ende gegenüberliegenden distalen Ende fixiert wird und eine vorgespannte zurückgezogene Position mit einem zylindrischen Profil, eine Außenfläche mit einer Schleifbeschichtung darauf und scharfe Schneidseitenkanten (104) umfasst, wobei die länglichen, flexiblen Elemente (100) mit der Antriebswelle (10) drehbar sind; eine mit der Antriebswelle wirkverbundene und mit dieser drehbare und mit den proximalen Enden aller flexiblen Elemente in der Vielzahl von länglichen, flexiblen Elementen (100) fest verbundene, proximale Halterung;
eine von der proximalen Halterung axial getrennte und mit den distalen Enden aller flexiblen Elemente (100) in der Vielzahl fest verbundene und mit der Antriebswelle (10) und der Vielzahl länglicher, flexibler Elemente (100) drehbare, distale Halterung; und
einen distalen, ein proximales fixiertes zylindrisches Profil und ein distales fixiertes konisches Profil, umfassenden sich verjüngenden Abschnitt (102), wobei der distale sich verjüngende Abschnitt (102) an der distalen Halterung befestigt ist und mit der Antriebswelle (10) und der Vielzahl länglicher, flexibler Elemente (100) drehbar ist;
wobei, wenn eine Verringerung der axialen Trennung der proximalen und distalen Halterung erfolgt, jedes Element (100) in der Vielzahl von der Antriebswelle (10) nach außen gebogen ist, und sich radial weg von der vorgespannten zurückgezogenen Position zu einer ausgedehnten Position ausdehnt, wodurch die flexiblen Elemente (100) ein ausgedehntes elliptisches Profil umfassen, **dadurch gekennzeichnet, dass**
die Antriebswelle (10) eine Drehachse umfasst, und die Vielzahl von flexiblen Elementen (100) ferner einen Massenmittelpunkt umfasst, der von der Drehachse durch eine asymmetrische Anordnung der flexiblen Elemente um die Antriebswelle oder ein oder mehrere flexible Elemente herum versetzt ist, umfassend ein Material mit einer anderen Dichte als die verbleibenden flexiblen Elemente;
so dass die ausgedehnte Vielzahl von flexiblen Elementen (100) einen ausgedehnten Ruhedurchmesser und einen rotierenden Schneiddurchmesser aufweist, wobei letzterer während einer Hochgeschwindigkeitsrotation erzielt wird, wobei der rotierende Schneiddurchmesser größer als der ausgedehnte Ruhedurchmesser wegen orbitaler oder exzentrischer Bewegung des Rotationsschneiders ist;
dass der Nasenkonus (7A) ein abrasiver Nasenkonus ist;
und dass der distale sich verjüngende Abschnitt (102) ein Lumen hindurch aufweist.

2. Rotierende Atherektomievorrichtung nach Anspruch 1, wobei die Vielzahl von flexiblen Elementen (100) flexible Elemente umfasst, die asymmetrisch angeordnet sind.

3. Rotierende Atherektomievorrichtung nach Anspruch 1, wobei die flexiblen Elemente (100) Drähte sind.

4. Rotierende Atherektomievorrichtung nach Anspruch 3, wobei zumindest einer der Drähte einen kreisförmigen Querschnitt aufweist.

5. Rotierende Atherektomievorrichtung nach Anspruch 3, wobei zumindest einer der Drähte einen nichtkreisförmigen Querschnitt aufweist.

6. Rotierende Atherektomievorrichtung nach Anspruch 1, zusätzlich umfassend eine Betätigungsvorrichtung, welche dem Betreiber das Steuern der Verringerung der axialen Trennung der proximalen und distalen Halterung ermöglicht.

7. Rotierende Atherektomievorrichtung nach Anspruch 1, wobei die flexiblen Elemente (100) in einer parallelen Anordnung montiert sind und in einem Winkel voneinander beabstandet sind.

## Revendications

1. Dispositif d'athérectomie rotatif pour abraser un matériau occlusif dans la lumière d'un vaisseau sanguin, comprenant :
un arbre d'entraînement rotatif, allongé et flexible (10) ayant une extrémité proximale (12) et une extrémité distale opposée à l'extrémité proximale (12) ;
un cône de nez (7A) comprenant :
une pluralité d'éléments flexibles et allongés (100) à proximité de l'extrémité distale de l'arbre d'entraînement (10), chaque élément (100) dans la pluralité étant fixé à la fois à une extrémité proximale et à une extrémité distale opposée à l'extrémité proximale et comprenant une position rétractée inclinée ayant un profil cylindrique, une surface extérieure recouverte d'un revêtement abrasif, et des bords latéraux tranchants (104), dans lequel les éléments flexibles et allongés (100) peuvent tourner avec l'arbre d'entraînement (10) ; une monture proximale reliée de manière opérationnelle avec l'arbre d'entraînement et pouvant tourner avec celui-ci et reliée de manière fixe aux extrémités proximales de tous les éléments flexibles dans la pluralité d'éléments flexibles et allongés (100) ;
une monture distale séparée axialement de la monture proximale et reliée de manière fixe aux extrémités distales de tous les éléments flexibles (100) dans la pluralité et pouvant tourner avec l'arbre d'entraînement (10) et la pluralité d'éléments flexibles et allongés(100) ; et
une section effilée et distale (102) comprenant un profil cylindrique, fixe et proximal et un profil conique, fixe et distal, la section effilée et distale (102) étant fixée à la monture distale et pouvant tourner avec l'arbre d'entraînement (10) et la pluralité d'éléments flexibles et allongés (100) ;
dans lequel, lorsque la séparation axiale des montures proximale et distale est réduite, chaque élément (100) dans la pluralité s'incline vers l'extérieur à partir de l'arbre d'entraînement (10) et s'étend radialement à l'écart de la position rétractée et inclinée vers une position étendue, les éléments flexibles (100) comprenant ainsi un profil elliptique et étendu, **caractérisé en ce que**
l'arbre d'entraînement (10) comprend un axe de rotation, et la pluralité d'éléments flexibles (100) comprennent en outre un centre de gravité décalé par rapport à l'axe de rotation par l'intermédiaire d'un arrangement asymétrique des éléments flexibles autour de l'arbre d'entraînement ou l'un ou plusieurs éléments flexibles comprennent un matériau d'une autre densité que celui des éléments flexibles restants ;
si bien que la pluralité étendue d'éléments flexibles (100) présente un diamètre étendu restant et un diamètre coupant rotatif, ce dernier étant obtenue lors de la rotation à haute vitesse, le diamètre coupant rotatif étant plus large que le diamètre étendu restant en raison du déplacement orbital ou excentrique du dispositif de coupe rotatif ;
**en ce que** le cône de nez (7A) est un cône de nez abrasif ;
et **en ce que** la section effilée distale (102) présente une lumière à travers celle-ci.

2. Dispositif d'athérectomie rotatif selon la revendication 1, dans lequel la pluralité d'éléments flexibles (100) comprend des éléments flexibles qui sont agencés de manière asymétrique.

3. Dispositif d'athérectomie rotatif selon la revendication 1, dans lequel les éléments flexibles (100) sont des fils.

4. Dispositif d'athérectomie rotatif selon la revendication 3, dans lequel au moins l'un des fils présente une section transversale circulaire.

5. Dispositif d'athérectomie rotatif selon la revendication 3, dans lequel au moins l'un des fils présente une section transversale non circulaire.

6. Dispositif d'athérectomie rotatif selon la revendication 1, comprenant en outre un dispositif d'actionnement qui permet à l'opérateur de commander la réduction de la séparation axiale des montures proximale et distale.

7. Dispositif d'athérectomie rotatif selon la revendication 1, dans lequel les éléments flexibles (100) sont montés dans un réseau parallèle et sont espacés angulairement les uns des autres.
